(19) Europäisches Patentamt
European Patent Office
Office européen des brevets

(11) Publication number: **0 127 865**
**A1**

# (12) EUROPEAN PATENT APPLICATION

(21) Application number: 84106054.4

(22) Date of filing: 28.05.84

(51) Int. Cl.³: C 07 D 249/08
A 61 K 31/41

(30) Priority: 03.06.83 US 501031

(43) Date of publication of application:
12.12.84 Bulletin 84/50

(84) Designated Contracting States:
IT

(71) Applicant: SCHERING CORPORATION
2000 Galloping Hill Road
Kenilworth, New Jersey 07033(US)

(72) Inventor: Wright, John Jessen
691 Marigold Court
Evansville Indiana 47712(US)

(72) Inventor: Cooper, Alan Bruce
14 Aldom Circle
West Caldwell New Jersey 07006(US)

(74) Representative: Antony, Fritz, Dr. et al,
c/o Scherico Ltd. P.O. Box 601 Töpferstrasse 5
CH-6002 Lucerne(CH)

## (54) Novel triazolyl-substituted propane derivatives, their preparation and pharmaceutical compositions containing them.

(57) Disclosed herein are novel triazolyl-substituted propane compounds of the general formula I

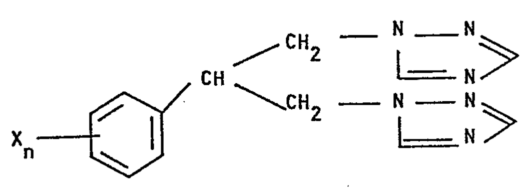

(I),

and the pharmaceutically acceptable salts thereof, wherein n is 1,2 or 3 and X independently represents halogen, lower alkoxy, lower alkyl or phenyl.

The novel compounds exhibit antifungal activity and their use for this purpose is disclosed. Also disclosed are methods for preparing the novel compounds as well compositions containing them.

EP 0 127 865 A1

NOVEL TRIAZOLYL- SUBSTITUTED PROPANE DERIVATIVES, THEIR PREPARATION AND PHARMACEUTICAL COMPOSITIONS CONTAINING THEM.

This invention relates to novel triazolyl-substituted propane derivatives useful as antifungal agents, to their preparation and to pharmaceutical compositions, for veterinary as well as human use, comprising the triazolyl compounds.

In its compound aspects, the present invention provides novel triazolyl-substituted propane derivatives of the general formula I:

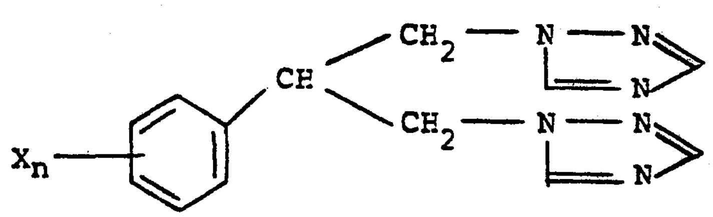

(I),

wherein n is 1, 2 or 3 and X independently represents halogen, lower alkoxy, lower alkyl or phenyl; and the pharmaceutically acceptable acid addition salts thereof.

./.

As used herein, the term "lower alkyl" means straight or branched alkyl chains having 1 to 6 carbon atoms, e.g. methyl, ethyl, n-propyl, n-butyl, iso-butyl, pentyl and hexyl. Similarly, "lower alkoxy" means straight or branched alkoxy groups having 1 to 6 carbon atoms, e.g. methoxy, ethoxy, propoxy, iso-propoxy and butoxy. The term "halogen" means chlorine, bromine or fluorine.

"Pharmaceutically acceptable acid addition salts" means those salts formed with inorganic or organic acids, e.g. hydrochloric, nitric, sulfuric, acetic, p-toluenesulfonic, phosphoric, maleic or oxalic acid.

Preferred are compounds wherein X represents halogen, preferably fluoro or chloro, and n is 1 or 2.

The compounds of formula I exhibit antifungal activity against such human and animal pathogens as Candida and Trichophyton, as demonstrated by conventional in vivo tests in animals, e.g. a mouse systemic infection model. These tests indicate the compounds of this invention to be orally active. In one such test (a chronic Candida kidney infection model in mice) mice, infected with Candida albicans, and orally treated with 100 mg/kg ketoconazole (a reference commercially available oral antifungal agent) per day for ten days and sacrificed about 5 days after treatment ended showed a geometric mean activity of 7 colony forming units, while infected mice similarly treated with 2-(4-chlorophenyl)-1,3-di-[1-(1H-1,2,4-triazolyl)]propane at levels of 50 mg/kg and 25 mg/kg showed geometric mean activities of 7.4 and 7.9 colony forming units, respectively. This indicates that 2-(4-chlorophenyl)-1,3-di-[(1H-1,2,4-triazolyl)] propane is approximately twice as potent as an antifungal gent as the reference compound.

. / .

Dosage level and mode of administration of the compounds of the present invention will vary in the judgement of the attending clinician according to the particular host and the type and severity of infection. In general, the dosage range will be from about 100 to about 500 mg per day, in single or divided doses, with the preferred range being about 125 mg to about 250 mg per day active ingredient, combined with a suitable pharmaceutically acceptable carrier or diluent. Regarding toxicity, the compounds of the formula I are non-toxic at the therapeutic dose.

In another of its aspects, the present invention provides a composition, especially for use as an antifungal, which comprises as active ingredient a compound of the formula I together with a pharmaceutically acceptable non-toxic carrier, preferably for oral administration. Such compositions cover both human and veterinary use. Typical oral dosage forms include tablets, capsules, elixirs, suspensions and the like. These dosage forms include typical pharmaceutically acceptable carriers, e.g. sugars such as lactose or sucrose, starches such as corn starch, cellulose and derivatives such as sodium carboxy cellulose or methyl cellulose, calcium phosphates, stearic acid, alkaline earth stearates such as magnesium stearate, and polyalkylene glycols. Other dosage forms include those suitable for topical use.

It is also contemplated that the antifungal compounds of this invention may be administered to animals in need of such treatment via a composition comprising the animal feed or drinking water conventionally used for the animal being treated.

The compounds of the general formula I may be prepared by methods known per se.

./.

In a first general method, the present invention provides a process for the preparation of the compounds of the general formula I which comprises reacting an alcohol of the general formula II:

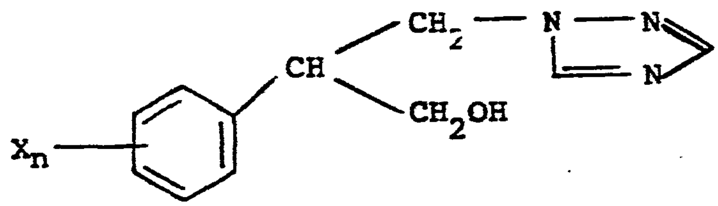

(II),

or a reactive derivative thereof, wherein n and X are as defined for formula I, with 1,2,4-triazole or a reactive derivative thereof and then isolating the so-obtained compound of the general formula I in free form or in the form of a pharmaceutically acceptable acid addition salt.

The reactive derivative of the alcohol of the formula II may be a reactive ester derivative such as a sulfonate ester of which the methyl sulfonate ester and trifluoromethylsulfonate esters may be mentioned as examples, or an ester of a hydrohalic acid, e.g. the chloride. The reactive derivative of 1,2,4-triazole may be a cationic salt, for instance an alkali metal salt such as the sodium salt.

Typically the reaction is effected in an inert medium, preferably in a solvent such as dimethylformamide or acetonitrile and at a temperature from about room temperature up to the reflux temperature of the reaction medium.

The starting material of the general formula II may be prepared in known manner by, for instance, reacting 1,2,4-triazole with an appropriate α-bromomethylstyrene in

the presence of sodium hydride to give the corresponding α-1(-1,2,4-triazolyl)methyl substituted styrene and then treating this product in manner known per se with borane in a suitable solvent such as tetrahydrofuran followed by oxidative treatment under alkaline conditions (e.g. treatment with hydrogen peroxide in the presence of a base such as sodium hydroxide) to give the corresponding alcohol of the general formula II. Typically, where the alcohol of the general formula II is employed in the form of a reactive ester derivative, that derivative may be prepared using conventional esterification techniques. Thus, for preparing a methanesulfonate ester derivative, the alcohol may be treated with a methanesulfonyl halide, such as the chloride, in a basic solvent such as pyridine. The reactive ester derivative may then be treated, if desired in situ, to give the desired compound of the formula I. Isolation of the product in free form or in the form of a pharmaceutically acceptable acid addition salt may be effected in known manner.

Another general method for the preparation of the compounds of the general formula I comprises reducing a substituted propene of the general formula III.

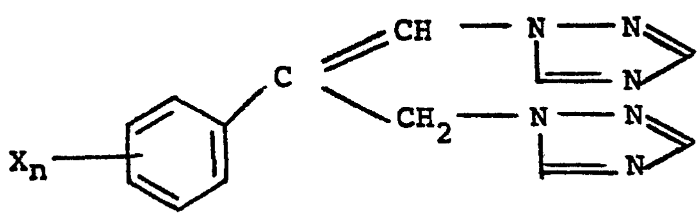

(III),

wherein n and X are as defined for formula I and then isolating the so-obtained compound of the general formula I

in free form or in the form of a pharmaceutically acceptable acid addition salt.

Reduction of the olefinic double bond may be effected in conventional manner using known hydrogenation techniques. Thus, we have effected hydrogenation using palladium on charcoal as the hydrogenation catalyst and glacial acetic acid as the inert reaction medium.

The starting material of the general formula III may be prepared in known manner by, for instance, reacting an X-substituted phenyl magnesium bromide with 1,3-dichloroacetone to give a 2-(X-substituted-phenyl)-1,3-dichloropropanol-2-ol which is then reacted with an appropriate alkali metal salt of 1,2,4-triazole to give a 2-(X-substituted-phenyl)-1,3-di-[1-(1H-1,2,4-triazolyl)]-propan-2-ol. This product is then converted to the desired substituted propene of the general formula III by treatment with thionyl chloride.

The invention will now be illustrated by way of the following Examples.

## EXAMPLE 1

## 2-(4-CHLOROPHENYL)-1,3-DI-[1-(1H-1,2,4-TRIAZOLYL)]-PROPANE

### A. 2-(4-chlorophenyl)-1-[1-(1H-1,2,4-triazolyl)]-2-propene

To 28 g. of a 60% sodium hydride-in-oil suspension (0.7 mol NaH) in 800 ml of dimethylformamide, add in portions 1,2,4-triazole (50 g, 0.72 mol) at -10 to 0°C. Stir at 0.5°C for 1 hour until the solution is clear brown. Add rapidly at -5 to 5°C 1-bromo-2-(4-chlorophenyl)-2-propene (200 g, 0.86 mol based on total bromide). Stir and warm to room temperature over 2 hours. Evaporate off the dimethylformamide. Add saturated aqueous sodium chloride solution and extract the product with 2 x 300 ml of ethyl acetate. Dry the combined extracts over magnesium sulfate and filter. Evaporate the ethyl acetate and chromatograph the resultant oil on 500 g of silica gel using ethyl acetate:hexane (1:1) as the eluent to obtain 13.2 g of 2-(4-chlorophenyl)-1-[1-(1H-1,2,4-triazolyl)]-2-propene 'HNMR (90 mHz, $CDCl_3$) δ 8.06 (1 H, S), 7.93 (1 H, S), 7.3 (H H, S), 5.59 (1 H, S), 5.16 (3 H, br S).

### B. 2-(4-chlorophenyl)-3-[1-(1H-1,2,4-triazolyl)propan-1-ol

Dissolve the product of part A (1 g, 4.5 mmol) in 30 ml of tetrahydrofuran and cool in an ice bath at 0°C under a static nitrogen atmosphere. Add 14 ml of a 1 molar solution of borane in tetrahydrofuran and let warm to room temperature. After 17 hours, destroy the excess borane with the addition of 5 ml of water. Add 10 ml of 3N sodium hydroxide and 10 ml of 30% hydrogen peroxide and let reflux. After two hours add saturated brine and extract with 3 x 50 ml of ethyl acetate. Dry the ethyl

acetate layers over magnesium sulfate, filter and evaporate to obtain a viscous syrup. Chromatograph on 100 g of silica gel using 5% methanol/chloroform as the eluant to obtain .44g (1.85 mmol, 41%) of the title compound.

C. 2-(4-chlorophenyl)-1,3-di-[1-(1H-1,2,4-triazolyl)]-propane

Dissolve the product of part B (.44 g, 1.85 mmol) in 10 ml of pyridine. Cool in an ice bath to $0^{o}C$ and add, dropwise, methanesulfonyl chloride (.17 ml, 2.20 mmol). Let slowly warm to room temperature and stir for 5 hours. Add 20 ml of water and extract with 3 x 50 ml of ethyl acetate. Dry the ethyl acetate layer over magnesium sulfate, filter and evaporate off the solvent to obtain a crude oil.

Dissolve the crude oil in 3.0 ml of N,N-dimethylformamide and add, dropwise, to a solution of sodium 1,2,4-triazole formed from the addition of triazole (.23 g, 3.40 mmol) in 3 ml of N,N-dimethylformamide to a mixture of sodium hydride (.08 g, 3.4 mmol) in 10 ml of N,N-dimethylformamide at $0^{o}C$. Warm and stir at $50^{o}C$ for two hours. Add 30 ml of water and extract with 3 x 50 ml of ethyl acetate. Dry the combined ethyl acetate layers over magnesium sulfate, filter and evaporate to dryness. Chromatograph the resulting oil on 50 g of silica gel using 5% methanol/chloroform as the eluant to obtain .20 g of the title compound 'H NMR(90mHz,$CDCl_3$) $\delta$ 8.0(2H,S), 7.93 (2H,S), 7.24(2H,d,J=7.5Hz), 6.9(2H,d,J=7.5Hz) 4.81-4.15 (4H,m), 4.07-3.68 (1H,m); M.S. m/e (FAB) 289 (100% $m^{+}41$)

EXAMPLE 2

2-(4-BIPHENYL)-1,3-DI-[1-(1H-1,2,4-TRIAZOLYL)]-PROPANE

A. 2-(4-biphenyl)-1,3-dichloro-propan-2-ol

To a mixture of 5.83 g of magnesium in 50 ml of diethyl ether, add dropwise over a period of 0.5 hours, 4-bromobiphenyl (51.26 g, 0.22 mol) dissolved in 250 ml of diethyl ether. Let the mixture reflux. After reaction is complete add to a solution of 1,3-dichloroacetone (25.2 g, 0.20 mol) in 270 ml of diethyl ether at -30°C. After 1.5 hours add 21 ml of glacial acetic acid in 100 ml of diethyl ether and let the mixture warm to 25°C. Add 200 ml of ethyl acetate and then wash with two 200 ml portions of water. Dry over magnesium sulfate, filter and then evaporate off the solvent to obtain a gum-like solid. Crystallise fromm 600 ml of hot hexane to obtain 63 g of the title compound m.p. 74-75°C.

B. 2-(4-diphenyl)-1,3-di-[1-(1H-1,2,4-triazolyl)]-propan-2-ol.

To a suspension of sodium hydride (9.6 g, 0.4 mol) in 50 ml of N,N-dimethyl formamide at 0°C, add dropwise, a solution of 1,2,4-triazole (27.6 g, 0.4 mol) in 50 ml of N,N-dimethyl formamide. After stiring for 1 hour add over 10 minutes a solution of 2-(4-biphenyl)-1,3-dichloro-propan-2-ol obtained in step A (28 g, 0.1 mol) in 50 ml of N,N-dimethylformamide. Stir at 100°C for 4 hours. Evaporate off the dimethylformamide at 50°C under vacuum. Add saturated aqueous sodium chloride solution and then extract the mixture with three 200 ml portions of ethyl acetate. Dry the ethyl acetate extract over magnesium sulfate, filter and then evaporate off the ethyl acetate at 50°C under

vacuum to obtain a solid product. Chromatograph on 500 g of silica gel using a mixture of 5% methanol in chloroform as eluant to obtain 10.2 g of the title compound. 'HNMR (90mHz,$CDCl_3$) $\delta$ 8.16(2H,S), 7.82 (2H,S), 7.6-7.25 (9H,m), 4.66(4H,S).

C. 2-(4-biphenyl)-1,3-di-[1-(1H-1,2,4-triazolyl)]-propane

To a solution of 3.46 g (10 m mol) of the product of step B in 25 ml of pyridine at $0^{o}C$, add 1.44 ml (20 m mol) of thionyl chloride. After stirring for 18 hours, add an aqueous saturated solution of sodium bicarbonate and then extract with two 50 ml portions of ethyl acetate. Dry the extract over magnesium sulfate, filter and then evaporate off the solvents to obtain an oil. Filter through 300 ml of silica gel washing with 5% methanol in chloroform. Evaporate the solvents at $50^{o}C$ under vacuum to obtain an oil again. Dissolve the oil in 100 ml of glacial acid and add 0.6 g of 10% palladium on charcoal. Hydrogenate at 50 p.s.i. of hydrogen for 8 hours. Filter off the catalyst and evaporate off the acetic acid. Chromatograph the product on 300 g of silica gel using 5% methanol in chloroform as eluant to obtain 2.5 g of the title compound: 'HNMR (90mHz,$CDCl_3$) $\delta$ 8.0(2H,S) 7.92 (2H,S), 7.68-7.27 (9H,m) 4.9-4.9 (4H,m), 4.2-3.72(1H,m).

In a similar manner to that described in Examples 1 and 2 one can prepare 2-(2,4-difluorophenyl)-1,3-di[1-(1H-1,2,4 -triazolyl)]-propane 'HNMR (90mHz,$CDCl_3$) $\delta$ 8.0(4H, superficial s), 7.1-6.5(3H,m),5.9-4.3(4H,m),4.4-4.0(1H,m).

Similarly, by using the appropriate starting materials the following compounds may be prepared:

2-(4-bromophenyl)-1,3-di-[1-(1H-1,2,4-triazolyl)]-propane;
2-(2,4-dichlorophenyl)-1,3-di-[1-(1H-1,2,4-triazolyl)]-propane;
2-(4-methylphenyl)-1,3-di-[1-(1H-1,2,4-triazolyl)]-propane;
2-(2,4-dimethylphenyl)-1,3-di-[1-(1H-1,2,4-triazolyl)]-propane;
2-(2-methyl-4-chlorophenyl)-1,3-di-[1-(1H-1,2,4-triazolyl)]-propane;
2-(2-methyl-4-fluorophenyl)-1,3-di-[1-(1H-1,2,4-triazolyl)]-propane;
2-(3,5-dichlorophenyl)-1,3-di-[1-(1H-1,2,4-triazolyl)]-propane;
2-(2-methoxy-4-fluorophenyl)-1,3-di-[1-(1H-1,2,4-triazolyl)]-propane;
2-(2-methoxy-4-methylphenyl)-1,3-di-[1-(1H-1,2,4-triazolyl)]-propane;
2-(2-methoxy-4-fluorophenyl)-1,3-di-[1-(1H-1,2,4-triazolyl)]-propane;
2-(2-fluoro-4-methylphenyl)-1,3-di-[1-(1H-1,2,4-triazolyl)]-propane,
2-(2-ethyl-4-methoxyphenyl)-1,3-di-[1-(1H-1,2,4-triazolyl)]-propane;

The following are typical pharmaceutical formulations containing as the active ingredient (designated "Drug") the compounds of this invention. The active ingredient may be 2-(4-chlorophenyl)-1,3-di-[1-(1H-1,2,4-triazolyl) propane or an equivalent amount of any of the other compounds of this invention.

FORMULATION 1

| Tablet | 125.00 mg. tab. |
|---|---|
| Drug | 125.00 mg. |
| Polyethylene glycol 6000 | 100.00 mg. |
| Sodium lauryl sulfate | 6.25 mg. |
| Corn starch | 30.00 mg. |

./.

FORMULATION 1 (Cont.)

| Tablet | 125.00 mg. tab. |
| --- | --- |
| Lactose, anhydrous | 87.25 mg. |
| Magnesium stearate | 1.50 mg. |

Procedure:

Heat the polyethylene glycol 6000 to 70-80°C. Mix the drug, sodium lauryl sulfate, corn starch, and lactose into the liquid and allow the mixture to cool. Pass the solidified mixture through a mill. Blend granules with magnesium stearate and compress into tablets.

FORMULATION 2

| Capsule | 250 mg capsule |
| --- | --- |
| Drug | 250.00 mg. |
| Lactose, anhydrous | 100.00 mg. |
| Corn starch | 50.00 mg. |
| Microcrystalline cellulose | 95.00 mg |
| Magnesium stearate | 5.00 mg. |

Procedure:

Mix the first four ingredients in a suitable mixer for 10-15 minutes. Add the magnesium stearate and mix for 1-3 minutes. Fill the mixture into suitable two-piece hard gelatin capsules using an encapsulating machine.

WE CLAIM:

1. Triazolyl substituted propane compounds of the general formula I:

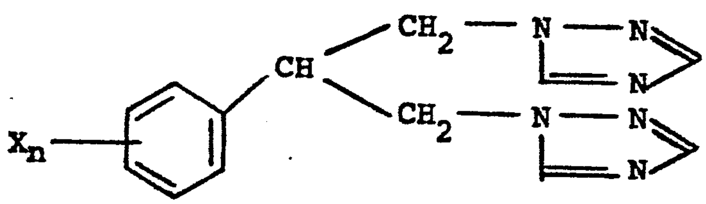

(I),

wherein n is 1, 2 or 3 and X independently represents halogen, lower alkoxy, lower alkyl or phenyl and the pharmaceutically acceptable acid addition salts thereof.

2. A compound as claimed in claim 1, wherein X is halogen, preferably fluorine or chlorine.

3. A compound as claimed in claim 1 or claim 2, wherein n is 1 or 2.

4. A compound as claimed in claim 3, said compound being selected from 2-(4-chlorophenyl)-1,3-di-[1-(1H-1,2,4-triazolyl)]propane and 2-(2,4-difluorophenyl)-1,3-di-[1-(1H-1,2,4-triazolyl)]propane.

5. A process for the preparation of a compound as claimed in claim 1, which process comprises one of the following processes A and B:

A. Reacting an alcohol of the general formula II:

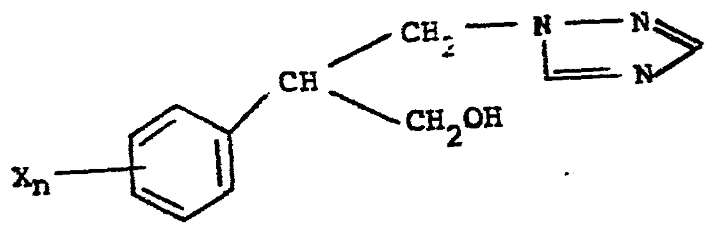

(II),

or a reactive derivative thereof, wherein n and X are as defined in claim 1, with 1,2,4-triazole or a reactive derivative thereof,

B. Reducing a substituted propene of the general formula III

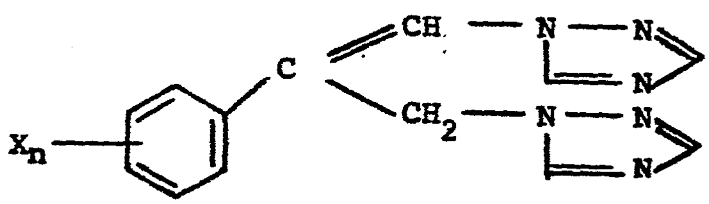

(III),

wherein n and X are as defined in claim 1, said process selected from processes A and B being followed by isolating the so-obtained compound in free form or in the form of a pharmaceutically acceptable acid addition salt.

6. A process as claimed in claim 5, wherein a reactive ester derivative of the alcohol of the general formula II is reacted with a cationic salt of 1,2,4-triazole, preferably an alkali-metal salt.

7. A process as claimed in claim 5, wherein reduction of the compound of the general formula III at the olefinic double bond is effected under hydrogen in the presence of suitable catalyst.

./.

0127865

8. A process as claimed in any one of claims 5 to 7, wherein X is halogen, preferably fluorine or chlorine.

9. A process as claimed in any one of claims 5 to 8 wherein n is 1 or 2.

10. A composition, especially for use as an antifungal, which comprises as active ingredient a compound as claimed in any one of claims 1 to 4 together with a pharmaceutically acceptable carrier.

11. A composition as claimed in claim 10 in a form suitable for oral administration.

Claims for Austria only:

1. A process for the preparation of a triazolyl substituted propane compound of the general formula I:

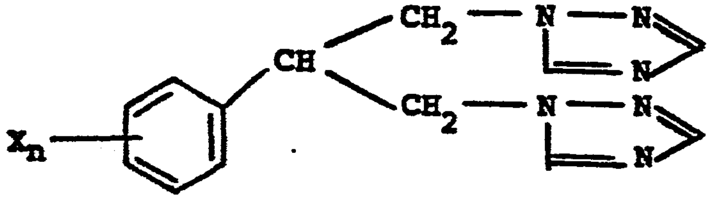

(I),

and the pharmaceutically acceptable acid addition salts thereof, wherein n is 1, 2 or 3 and X independently represents halogen, lower alkoxy, lower alkyl or phenyl, which process comprises one of the following processes A and B:

A. Reacting an alcohol of the general formula II:

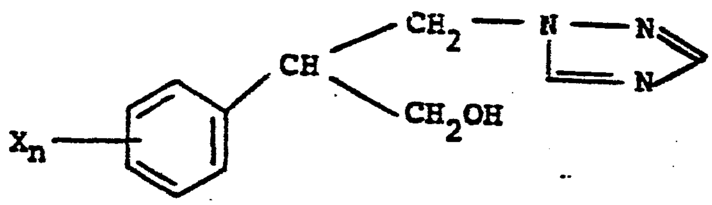

(II),

or a reactive derivative thereof, wherein n and X are as defined for formula 1, with 1,2,4-triazole or a reactive derivative thereof,

B. Reducing a substituted propene of the general formula III

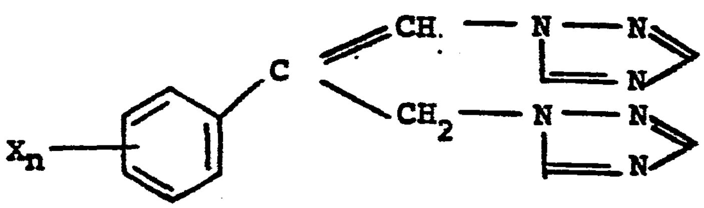

(III),

wherein n and X are as defined for formula 1, said process selected from processes A and B being followed by isolating the so-obtained compound in free form or in the form of a pharmaceutically acceptable acid addition salt.

2. A process as claimed in claim 1, wherein a reactive ester derivative of the alcohol of the general formula II is reacted with a cationic salt of 1,2,4-triazole, preferably an alkali-metal salt.

3. A process as claimed in claim 1, wherein reduction of the compound of the general formula III at the olefinic double bond is effected under hydrogen in the presence of suitable catalyst.

4. A process as claimed in any one of claims 1 to 3, wherein X is halogen, preferably fluorine or chlorine.

5. A process as claimed in any one of claims 1 to 4 wherein n is 1 or 2.

6. A process for the preparation of a composition, especially for use as an antifungal, which comprises admixing a compound of the general formula 1 set forth in claim 1 with a pharmaceutically acceptable carrier.

7. A process as claimed in claim 6, wherein the compound of the general formula 1 set forth in claim 1 has been prepared by a process as claimed in any one of claims 1 to 5.

European Patent Office

# EUROPEAN SEARCH REPORT

0127865
Application number

EP 84 10 6054

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 069 442 (PFIZER LTD.)<br>* the whole document *<br>----- | 1-3,10-11 | C 07 D 249/08<br>A 61 K 31/41 |
| | | | **TECHNICAL FIELDS SEARCHED (Int. Cl 3)** |
| | | | C 07 D 249/00 |

The present search report has been drawn up for all claims

| Place of search | Date of completion of the search | Examiner |
|---|---|---|
| THE HAGUE | 12-09-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document